# EUROPEAN PATENT APPLICATION

(11) **EP 4 124 291 A1**
(43) Date of publication of application: **01.02.2023**
(21) Application number: 21187880.6
(22) Date of filing: 27.07.2021
(51) Int. Cl.: A61B 5/0531, A61B 5/00, A61B 5/0533

(54) **DETECTION OF SUBJECT RESPONSE TO MECHANICAL STIMULI IN THE MOUTH**

(71) Applicant: Koninklijke Philips N.V., 5656 AG Eindhoven (NL)
(72) Inventor: KOOIJMAN, Gerben, 5656 AE Eindhoven (NL); GERHARDT, Lutz Christian, 5656 AE Eindhoven (NL); HIWALE, Sujitkumar, 5656 AE Eindhoven (NL); JOHNSON, Mark Thomas, 5656 AE Eindhoven (NL); SPELT, Hanne Adriana Alijda, 5656 AE Eindhoven (NL)
(74) Representative: Philips Intellectual Property & Standards

(57) **Abstract**

A method and system for monitoring an oral health indicator of a subject at one or more sites in the mouth via measurement of their pain response to applied mechanical stimuli at the one or more sites. One set of embodiments comprises determining an oral health indicator for a plurality of sites in the mouth based on application of mechanical stimuli to the different sites and based on recording a pain response of the subject to each stimulus, where the pain response is objectively measured via a physiological signal which is a known proxy indicator of arousal. At least one set of embodiments comprises a system which includes an oral tool for use in applying a standardized mechanical stimulus to an oral location in the mouth of a subject.

## Description

### FIELD OF THE INVENTION

This invention relates to a method for monitoring a subject's pain response to mechanical stimuli during an oral care session.

### BACKGROUND OF THE INVENTION

Many oral pathologies are associated with a pain response in the patient responsive to contact or other mechanical stimulation applied to the affected area.

For example, periodontitis is a chronic gum disease affecting approximately 46% of the adult US population, yet more than 50% of those affected are undiagnosed. A pain response on probing is plausibly assumed to be proportional to the periodontal pocket depth and thus to the degree of periodontal disease (V. Canakci & C. F. Canakci, "Pain levels in patients during periodontal probing and mechanical non-surgical therapy", Clin Oral Invest (2007) 11:377-383). For example, according to this paper, subjectively reported pain levels (using the Visual Analogue Scale (VAS) method) during probing were significantly higher in sites for which pocket depth is greater than 4 mm compared to sites with pocket depth of less than 4 mm deep. In addition, sites bleeding on probing had significantly higher VAS scores than sites with no bleeding on probing. Thus, for periodontitis, it can be seen that pain response is closely correlated with disease severity.

Furthermore, other oral pathologies are also associated with pain. For example a tooth infection results in pain upon application of pressure to the affected tooth, and wherein the severity of infection may be associated with the intensity of the pain response. Furthermore, other gum diseases such as gingivitis or even oral ulceration are associated with a pain response correlated with disease severity.

It is known that certain biological/physiological signals can change in dependence upon a patient pain response to a physical stimulus. One such biological signal is skin conductance which can be measured using galvanic skin resistance. Skin conductance measurements reflect changes in resistance and electrical conductivity in the skin as result of physiological arousal that can indicate acute pain. Another example is heart rate or heart rate variability. Physiological measures provide an objective quantification of pain and/or other arousal responses.

### SUMMARY OF THE INVENTION

The general concept proposed herein is to use the pain response of a subject to detect and track oral disease in subjects. Although the pain response is known to be associated with oral disease, it has not previously been proposed to use this to provide an objective, comparable metric for disease severity. Furthermore, known methods for measuring pain response in oral care are based on subjective reporting by the patient. A common method for measuring the level of pain is for example the Visual Analogue Scale (VAS). This is however highly subjective. It is also non-automatized. Furthermore, the scale is typically employed only once, after dental treatment has finished, and thus this method of pain measurement is useful only for measuring relative changes in the pain of a given patient over multiple different sessions.

The invention is defined by the claims.

According to examples in accordance with an aspect of the invention, there is provided a processor for use in measuring a subject pain response to a standardized mechanical stimulus applied using an oral tool at a plurality of locations in a subject's mouth, and for assessing a pre-determined oral health indicator at the plurality of locations based thereon.

The processor is adapted to: receive a biological/physiological signal of the subject; obtain an indication of position of the oral tool for each of a plurality of mechanical stimuli applied; determine from the biological signal a subject pain response to each applied mechanical stimulus; determine (e.g. using an algorithm) an oral health state indicator for each mouth location based on the pain response associated therewith; and generate a digital record comprising a representation of the oral health state indicator as a function of mouth location.

The problem to be solved is to enable an objective, systematized assessment of an acuity of one or more oral health conditions, such as periodontal disease, at different locations in the mouth. A pain level experienced by a subject responsive to a reference physical interaction/engagement by a tool with an oral surface is used as a proxy measure for disease severity.

In some cases, the oral health state indicator may simply be a measure of a pain level experienced by the subject responsive to the mechanical stimulus applied to the given location. Thus, the record might simply recite the pain level as a function of location in the mouth. This provides an indirect representation of a health state at each location. Alternatively, an algorithm or transfer function might be applied to convert the pain information (from the biological signal) into a metric for a particular oral health condition such as periodontal disease.

A pre-determined mechanical stimulus is applied at each of a plurality of oral sites and pain response measured. For example, the mechanical stimulus may be insertion of an oral probe into periodontal pockets at tooth sites around the mouth, where the insertion is done with a pre-defined force or depth level. Other stimuli could include insertion of probing members in interdental spaces, or the application of a tapping force to an exposed surface of a tooth or to the gum.

The biological/physiological signal is a signal which is directly or indirectly correlated with patient psychological arousal.

In some embodiments, the oral health state indicator may be an indicator of a severity of periodontal disease.

In some embodiments, the mechanical stimulus may comprise application of a standard reference force to an oral surface.

In some embodiments, the mechanical stimulus may comprise probing a periodontal pocket to a standard reference depth.

In some embodiments, the mechanical stimulus may comprise a temperature stimulus.

The obtaining of the indication of the position of the oral tool for a given mechanical stimulus may comprise: receiving a positioning signal from a position tracking apparatus indicative of position of the oral tool; obtaining an indication of a location indicated in a prompt delivered to a user by a user interface for prompting the user to manually apply the stimulus at the indicated location; or receiving from a user interface a user-input indication of the position of the oral tool.

In some embodiments, the biological signal may comprise a skin conductance signal received from a skin conductance sensing apparatus. The skin conductance sensing apparatus may be a galvanic skin response measurement device. Other examples of suitable biological signals may include heart rate, PPG signal, pupil dilation.

In some embodiments, the processor may be further adapted to generate user feedback via control of a user interface device operatively coupled to the processor. The user feedback may comprise a visual representation of the oral health state indicator as a function of mouth location.

In some embodiments, the processor may be adapted to access a health record datastore, and to append the generated digital record of the oral health state indicator as a function of mouth location to the datastore, associated with subject identification information, and associated with a time-stamp. This allows a record to be kept of the oral disease state of the subject across locations within the mouth over time.

In some embodiments, the processor may be adapted to access the health record datastore, to access historical records stored in the datastore for the subject of the oral health state indicator as a function of mouth location, and to compare the determined health state indicator for one or a plurality of the mouth locations with the historical values. For example, the health state indicator may be a measure which is relative in nature, and therefore a change in the value of the indicator over time may provide the most relevant information.

In some examples, the processor may be adapted to generate longitudinal trend information for the oral health state indicator for one or a plurality of locations in the mouth and to output the trend information to a user interface for presentation to a user.

In some embodiments, the oral health state indicator may be a graded metric indicative of a severity level of an oral condition or disease, and wherein the graded metric for each mouth location is computed in dependence upon a determined pain level at the location. Alternatively, the oral health state indicator may be a binary metric indicative of presence or absence of an oral disease.

A further aspect of the invention provides an oral tool for use in applying a standardized mechanical stimulus to an oral location in the mouth of a subject, wherein the tool includes indicator or feedback means for guiding a manual application of the stimulus by a user, or the tool includes an actuation mechanism for automatically applying the stimulus.

A further aspect of the invention provides a system comprising the aforementioned oral tool, and further comprising a means for measuring a biological signal directly or indirectly indicative of a subject pain response to each applied stimulus.

The system may further comprise a processor adapted to obtain an indication of the subject pain response to each of a plurality of mechanical stimuli applied using the oral tool at different locations in the mouth over a session and based on (e.g. processing of) the biological signal. The processor may further be adapted to determine, for example using a classifier algorithm, an oral health state indicator associated with each pain response, and to generate a digital record thereof.

The subject pain response may be obtained by: the processor receiving the biological signal and determining each pain response based thereon; or the processor receiving via a user interface a user-input indication of the pain response, obtained by the user manually from the biological signal.

In some embodiments, the processor may be further adapted to obtain an indication of a mouth location associated with each pain response and wherein the digital record represents the oral health state indicator as a function of mouth location.

In some embodiments, the oral tool may comprise a powered actuation mechanism (e.g. electrical or pneumatic) for applying the mechanical stimulus. Here, the tool comprises a powered mechanical stimulus generator actuable to apply a defined mechanical stimulus to an oral location.

By way of one example, where the tool is a powered toothbrush, the actuation mechanism might comprise driving a controlled tapping action by the protruding cleaning elements of the toothbrush head. This might be a cyclical tapping motion in some examples. In further examples, the mechanical stimulus may comprise a defined excursion or travel path of cleaning elements of the toothbrush over an oral surface. Alternatively, the tool may be a mouthpiece which comprises actuable probing or urging elements for applying stimuli at defined oral locations when the mouthpiece is received in the mouth.

In some embodiments, the oral tool may comprise a dental probe for probing a periodontal pocket with a probe tip. The indicator or feedback means may comprise a probing depth indicator which provides mechanical or visual feedback to an operator of the probe indicative of when the probe tip has been inserted to a predetermined depth in the periodontal pocket.

In one set of examples, the probing depth indicator may comprise a ridge or outward protrusion from a surface of the probe tip, at a height from a terminal distal point of the probe tip corresponding to the pre-determined depth in the pocket to be probed.

In some embodiments, the oral tool may comprise a dental probe for probing a periodontal pocket with a probe tip, and wherein the dental probe comprises a resilient element physically coupled with the probe tip for providing a resistive force feedback indicative of when a force with which the probe tip is being applied to an oral location by a user is equal to a pre-determined reference force.

The resilient element may provide a force limiter. It may have the effect of imposing an effective maximum applicable force, to thereby ensure the user of the dental probe applies a consistent reference force stimulus. It may comprise a spring member. It may provide a spring suspension mechanism.

In some embodiments, the oral tool may comprise a probe portion for insertion in interdental spaces, and/or for application on interdental papilla, and optionally wherein the probe portion comprises a fork member with at least two prongs.

For example, the pair of prongs may be for insertion in the interdental spaces either side of a tooth. The spacing between the prongs may be adjustable, e.g. they may be resiliently displaceable from one another.

In some embodiments, the system may comprise a biological signal measurement apparatus. In some embodiments, the biological signal measurement apparatus may be a skin conductance measurement apparatus.

In some embodiments, the tool may be an oral irrigator or powered flosser, and wherein the mechanical stimulus comprises a controlled pulse of fluid from a fluid jet.

A further aspect of the invention provides a computer-implemented method for use in measuring a subject pain response to a standardized mechanical stimulus applied using an oral tool at a plurality of locations in a subject's mouth, and for assessing a pre-determined oral health indicator at the plurality of locations based thereon, the method comprising: receiving a biological/physiological signal of the subject (which is preferably correlated with subject psychological arousal); obtaining an indication of position of the oral tool for each of a plurality of mechanical stimuli applied; determining from the biological signal a subject pain response to each applied mechanical stimulus; determining an oral health state indicator for each mouth location based on the pain response associated therewith; and generating a digital record comprising a representation of the oral health state indicator as a function of mouth location.

A further aspect of the invention provides a computer program product comprising code means configured to cause a processor to perform the method recited above, or in accordance with any embodiment described in this disclosure or any claim, and in particular when the processor is operatively coupled with a biological signal sensing means for measuring a biological parameter of the subject, directly or indirectly indicative of a pain response of the subject to each mechanical stimulus.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an example processor in accordance with one or more embodiments;
Fig. 2 shows a block diagram of an example system according to one or more embodiments of the invention; and
Figs. 3-5 illustrate analysis of temporal proximity (or temporal correlation) between contact events and physiological response events for differentially detecting a pain response.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for monitoring an oral health indicator of a subject at one or more sites in the mouth via measurement of their pain response to applied pre-determined mechanical stimuli at the one or more sites in the mouth. For example, one set of embodiments comprises determining an oral health indicator for a plurality of sites in the mouth based on application of mechanical stimuli to the different sites and based on recording a pain response of the subject to each stimulus, where the pain response is objectively measured via a physiological signal which is a known proxy indicator of emotional/psychological arousal. At least one set of embodiments comprises a system which includes an oral tool for use in applying a standardized physical stimulus to an oral location in the mouth of a subject.

Thus at least one aspect of the general inventive concept is a means for generating standardized, reproducible physical/mechanical stimuli, such that variation in the measured pain responses at different sites can be reliably assumed correlated with the health indicator and not differences in the stimuli. In this way, pain response of the subject can be reliably used as a proxy for the oral health state indicator.

At least one further aspect of the invention comprises measurement of pain response at a plurality of locations based on use of a biosignal such as skin conductance or heart rate, and generating a data record of the health indicator status as a function of location in the mouth. This may be referred to as a digital mouth map.

The oral heath state indicator may be a diagnostic indicator.

The oral health indicator may be an indicator of a diagnostic state of a particular oral disease such as periodontitis. It may be a binary indicator (diseased / not diseased) or it may be a graded metric, e.g. a level of disease along a graded scale. The latter is preferred since it allows differential analysis of the spread of disease as a function of location in the mouth at any one time, and also allows for tracking of disease progression over time. For example, in the case of periodontal disease, it can be used to detect and track classification of each site according to the standardized four-stage progression model.

The proposed method can be embodied in systems for home use by a patient, due to the objective and automated means by which the pain response and corresponding health state indicator are determined and recorded. Real time expert analysis is not required to obtain the health state indicator. The proposed method can also be embodied in systems for professional clinical use. The proposed method reduces the effort and time needed by a dental practitioner to conduct an examination and the duration of pain experience for the patient is shorter. It also allows the dental professional a more convenient and reliable way to track disease progression.

In embodiments, a biological signal of the subject is used to measure a pain response of the subject to applied mechanical stimuli. In one set of embodiments, skin conductance measurement is used as the biological signal. A brief introduction to skin conductance measurement will now be outlined.

Skin conductance is mediated through electrodermal activity, i.e. sweating. This has been shown to be linked to pain experiences (see e.g. H. Storm, "Changes in skin conductance as a tool to monitor nociceptive stimulation and pain," Curr. Opin. Anaesthesiol., vol. 21, no. 6, pp. 796-804, 2008.)

The electrodermal system behaves uniformly across the body. However, the eccrine palmar and plantar sweat glands are especially responsive to psychological stimuli, such as pain. High skin conductance levels and peaks in the skin conductance signal, reflect emotional arousal, whereas low levels and smooth signals reflect relaxation and comfort (see e.g. S. D. Kreibig, "Autonomic nervous system activity in emotion: A review," Biol. Psychol., vol. 84, no. 3, pp. 394-421, 2010.).

In painful situations, the electrodermal system becomes activated and the sweat glands fill up. This causes a reduction in skin resistance, and skin conductance consequently increases. Shortly after, the values return to their previous levels. This produces an identifiable peak in the skin conductance signal (which may be referred to as an electrodermal response, EDR), and the amplitude of the peak illustrates the firing strength of the sympathetic nerve, which is in turn correlated with acute pain level. These peaks are stimulus-specific, appear after a short time delay (typically around 1-2s), and fall off after the event.

Skin conductance can be measured via galvanic skin response (GSR) measurement equipment.

Skin conductance can show both psychological stress and pain. Pain and non-pain arousal events can be detected by analyzing the skin conductance signal, and identifying relevant peaks in the signal. A relevant peak might be identified for example based on the sum of the height of the leading edge in the peak, or the area under the peak curve during a certain time interval. Further metrics of the phasic and tonic components of skin conductance can be found in: J. T. Cacioppo, L. G. Tassinary, and G. G. Berntson, The handbook of psychophysiology, 3rd ed., vol. 44. New York: Cambridge University Press, 2007; and also in W. Boucsein, Electrodermal activity, 2nd ed. New York, NY: Springer Science+Business Media, 2012.

Skin conductance is not the only biological signal which is correlated with pain. For example, heart rate, PPG signal, eye movement, or pupil dilation may also be correlated with emotional arousal. For example, for heart rate, a data series of heart rate values as a function of time may be measured, and wherein peaks in the heart rate are used in place of peaks in skin conductance for detecting pain and/or non-pain arousal events.

Various embodiments may comprise a combination of one or more of the following components which together contribute to the general inventive concept.

Some embodiments may comprise a means (e.g. in the form of an oral tool) for providing a standardized and reproducible physical stimulus to one or more locations in the mouth, e.g. a tooth, tooth site, or tooth site pocket.

Some embodiments may comprise a means for obtaining an indication of at which location each stimulus is provided. This may be a location sensing or detecting means. This may alternatively be provided by user-input through a user interface, or by reference to a pre-determined location schedule, and optionally wherein prompts may be provided to a user indicative of each next location.

Some embodiments may provide a means for sensing a patient physiological response to each physical stimulus. This may comprise a biological signal sensing apparatus or device.

Some embodiments may provide an algorithm for relating the pain response to the health state indicator (e.g. a graded degree of severity of a particular oral disease) at the stimulus location. This may in some cases comprise determining an absolute measurement of a health state, or may comprise a relative metric (by comparison with previous values for the subject). In some cases, it may be provided simply by a measured level of the pain response.

Determining the oral health state indicator for each location may in some examples comprise accessing a pre-stored look-up table which relates measured pain response (e.g. pain level) with a corresponding value for the oral health state indicator, e.g. severity of disease.

In some examples, determining the oral health state indicator for each location may comprise application of a transfer function, of which the measured pain response (e.g. pain level) is an independent variable, and wherein evaluation of the function using the measured pain response as an input provides an output value for the oral health state indicator.

In some examples, determining the oral health state indicator may comprise use of an algorithm (e.g. a classifier algorithm), adapted to obtain a value of a health state indicator based on the measured pain response. The algorithm might be a classical algorithm, or might be a pre-trained machine learning algorithm in some examples.

Some embodiments may further comprise an algorithm for constructing a digital mouth map, indicating the health metric, or the relative degree of pain, or an indicator of health state progression over time, as a function of location in the mouth.

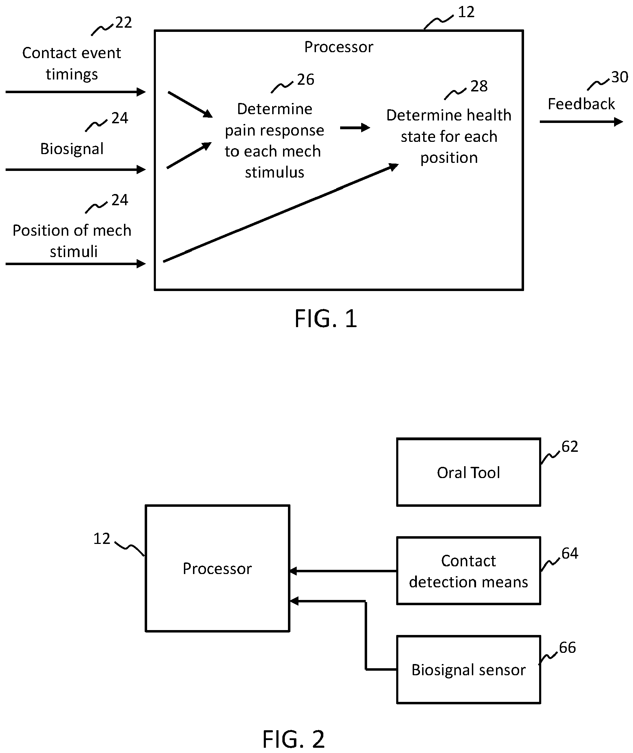

An example processor in accordance with one or more embodiments of the invention is shown in Fig. 1.

The processor 12 is for use in measuring a subject pain response to a standardized mechanical stimulus applied using an oral tool at a plurality of locations in a subject's mouth, and for assessing a pre-determined oral health indicator at the plurality of locations based thereon.

The processor is adapted to receive a biological signal 24 of the subject. This may be a skin conductance signal for example, or a heart rate signal.

The processor is further adapted to obtain an indication 24 of an oral position of the oral tool for each of a plurality of mechanical stimuli applied.

The processor is further adapted to determine from the biological signal a subject pain response to each applied mechanical stimulus. This may be based on detecting a peak or spike in the biological signal temporally correlated or synchronous with the applied stimulus, e.g. occurring within a pre-defined time window following the application of the mechanical stimulus. It may be based on detecting such a peak or spike for which the leading edge exceeds a predetermined height.

The processor is further adapted to determine an oral health state indicator for each mouth location based on the pain response associated therewith, for example based on use of a classifier algorithm.

The processor is further adapted to generate a digital record comprising a representation of the oral health state indicator as a function of mouth location. This may be exported to a datastore for example. For example, the processor may be adapted to access a health record datastore, and to append the generated digital record of the oral health state indicator as a function of mouth location to the datastore, associated with subject identification information, and associated with a time-stamp. This information can be used to track progression of the health state indicator as a function of time.

For example, the same processor, or a different processor, may be adapted to access the health record datastore, to access historical records stored in the datastore for the subject of the oral health state indicator as a function of mouth location, and to compare the determined health state indicator for one or a plurality of the mouth locations with the historical values. Longitudinal trend information for the oral health state indicator for one or a plurality of locations in the mouth may be determined. The trend information may be output to a user interface for presentation to a user.

With regards to the detecting of the pain response, this may be done in the following way.

The processor may further be adapted to receive, during the measurement session, the biological signal for the subject from a biological signal sensing apparatus coupled to the subject. The processor may be adapted to identify physiological response events in the biological signal. A physiological response may mean a spike in the signal up or down, e.g. peak or drop in the signal. For example, a physiological response event may be characterized by an event fingerprint in the biological signal comprising a spike in the signal having one or more pre-defined characteristics. For example, the characteristics may include a peak or drop height relative to a baseline exceeding a pre-defined threshold, or a leading edge exceeding a pre-defined height threshold. The characteristics may include spectral characteristics, e.g. a spectral signature of the peak or spectral components of the peak/drop. The baseline may be defined by a baseline measurement of the biological signal performed before the examination or measurement session begins, preferably with the subject at rest.

In some cases, the processor may be adapted to obtain an indication of timings of application of the mechanical stimuli, where each application of a mechanical stimulus may be referred to as a contact event. The biological signal may be probed or sampled subsequent to each contact event to detect any physiological response and then to classify whether and/or to what extent there is an identifiable pain signature in the physiological response. Based on the detected pain response, the health state indicator can be determined. Classifying a physiological response as a pain event may depend for example on the associated peak or drop in the signal having a leading edge which exceeds a pre-determined height relative to a baseline, or a sum of values along the leading edge of the peak or drop exceeding a pre-defined threshold, or an area under the peak or drop exceeding a pre-defined threshold. A pain level may be determined, for example based on a height of the peak or drop, or height of the leading edge of the peak or drop.

In some examples, the processor may further be adapted to determine a temporal proximity between each physiological response event and an immediately preceding contact event, and to classify the physiological response event as a pain event only contingent on the temporal proximity meeting a pre-defined criterion. For example, the processor may be configured to classify a physiological response event as associated with a pain state dependent on the physiological response event occurring within a pre-defined time window immediately following a contact event.

The indication of the timings of the contact events (the mechanical stimuli) might be obtained from one of: a contact detection means which actively senses when each mechanical stimulus is applied to an oral surface (e.g. integrated in the oral tool); a user interface which receives a user-input signal each time the user applies a mechanical stimulus (e.g. input by an assistant or using a button or pedal); a set of prompts issued to a user interface prompting a user to apply a stimulus responsive to generation of the prompt (where the prompts may be visual indications on a screen or audible sounds, or haptic signals issued through the tool).

The obtaining of the indication of the position of the oral tool for a given mechanical stimulus can be done in different ways. In some examples, it may comprise receiving a positioning signal from a position tracking apparatus indicative of position of the oral tool. In some examples, it may comprise obtaining an indication of a location indicated in a prompt delivered to a user by a user interface for prompting the user to manually apply the stimulus at the indicated location. In some examples, it might comprise receiving from a user interface a user-input indication of the position of the oral tool.

Although Fig. 1 shows a single processor, the steps carried out thereby may be implemented by a processing arrangement comprising multiple processors, or may be implemented by a distributed processing system, e.g. a cloud based processing system. The processor may be operatively coupled to an input/output (I/O) via which the input signals are received and the data outputs are exported.

Fig. 2 shows components of an example system, some or all of which may be provided in accordance with embodiments of the invention.

The system comprises an oral tool 62 for use in applying a standardized mechanical stimulus to an oral location in the mouth of a subject. The tool may include indicator or feedback means for guiding a manual application of the stimulus by a user. Alternatively the tool may include an actuation mechanism for automatically applying the stimulus. The actuation mechanism may be powered (e.g. electric, or pneumatic), or may be non-powered (e.g. elastically charged for each actuation event).

One aspect of the invention provides the oral tool alone. Example implementations of the oral tool will be described later in this disclosure in the context of example systems. It is to be noted that in each case the tool could be provided by itself as a separate aspect of the invention.

The system may further comprise a means 66 for measuring a biological signal directly or indirectly indicative of a subject pain response to each applied stimulus. This may in some examples comprise a skin conductance sensing apparatus such as a Galvanic Skin Response measurement apparatus.

The system may further comprise a processor 12 adapted to obtain an indication of the subject pain response to each of a plurality of mechanical stimuli applied using the oral tool 62 at different locations in the mouth over a session, and to determine using a classifier algorithm an oral health state indicator associated with each pain response, and to generate a digital record thereof. The subject pain response may be obtained by: the processor receiving the biological signal and determining each pain response based thereon; or the processor receiving via a user interface a user-input indication of the pain response, obtained by the user manually from reading of the biological signal.

The processor 12 may be further adapted to obtain an indication of a mouth location associated with each pain response and wherein the digital record represents the oral health state indicator as a function of mouth location.

In certain examples, the system may further comprise contact detection means 64 for detecting application of each mechanical stimulus. As discussed above, this could be a sensor for detecting each contact event (each mechanical stimulus). Alternatively, it could be a user interface which receives a user-input each time a mechanical stimulus is applied. Alternatively, it could be a signal from a user interface controller which generates prompts guiding a user as to timings of each new stimulus.

Two illustrative embodiments will now be outlined in detail: a first for clinical use, and a second for home use by a user.

According to a first embodiment, a system is provided which comprises means for providing a stimulus at a tooth, tooth site, or tooth site pocket. This may be for use in a clinical setting by a dental professional (DP).

The means for providing the stimulus may be an oral tool used by the dental practitioner. According to one set of examples, the oral tool may comprise a dental probe for use in probing periodontal pockets. It may comprise a handle leading to a probe tip at a distal end. Its structure in this respect may be similar to that of standard dental probes currently used for periodontal probing. However, the probe may be adapted such that it permits application of a standardized mechanical stimulus to an oral location in the mouth of a subject. This could be through an indicator or feedback means for guiding a manual application of the stimulus by a user. Alternatively, it could be through inclusion of an actuation mechanism for automatically applying the stimulus.

By way of one example, the probe may be provided with a ridge or outward protrusion from a surface of the probe tip, at a height from a terminal distal point of the probe tip corresponding to the pre-determined depth in the pocket to be probed. The DP inserts the probe into a periodontal pocket until the ridge touches the gum: this will ensure that each pocket is probed to the same depth according to the patient's demographic group (e.g. based on age, gender). In this way a uniform stimulus is provided to each tooth site pocket, where the stimulus in this case is probing of the periodontal pocket to a pre-defined depth.

According to a second example, the dental probe may be provided with a resilient element physically coupled with the probe tip for providing a resistive force feedback indicative of when a force with which the probe tip is being applied to an oral location by a user is equal to a pre-determined reference force. Thus, in contrast with the previous example which was based on guiding probing to a defined probing depth, this example aims to guide a user in applying a fixed probing pressure.

The oral tool may be a dental probe modified to limit the pressure during probing. This may include a spring-mounted probe tip, whereby the probe tip pressure is limited by the spring suspension (the tip resiliently retracts into the body of the tool responsive to pressure, with resilient biasing means biasing it back toward an exposed position). Another approach is to dynamically limit the pressure using an active actuation element, such as a responsive material (such as an electro-active polymer). The DP inserts the probe into a pocket until the pressure limitation is reached according to the patient's demographic group: this will ensure that each pocket is probed with the same probe pressure. In this way a fixed stimulus is provided to each tooth site pocket with the same probing pressure.

Thus the tool provides a device for generating a standardized and reproducible physical stimulus. This allows for reliably correlating pain response to oral health indicator status. For example it avoids inadvertent differences in the intensity of the stimulus, for example due to variation in access to the teeth (upper/vs lower, left vs right, front mouth vs rear mouth), or fatigue or error of the DP (e.g. more consistent at the start of the examination, less consistent at the end).

The system in this embodiment further comprises means for obtaining an indication of a location in the mouth at which the stimulus is provided. This can be achieved in different ways. One example is to use location sensing. For instance, the probe (or other device for providing the mechanical stimulus) may be comprise one or more integrated sensors for use in location detection, such as an Inertial Measurement Unit (IMU), camera, or other optical sensors (proximity or distance sensors). The probe may additionally or alternatively be part of a location sensing system, comprising at least an additional unit external to the probe: for instance, a fixed camera tracking the probe position relative to the patient, or a transceiver arrangement with an electromagnetic or other modality emitter spaced from the probe, and the probe having a transceiver for redirecting the received signal back (or vice versa). Another example is use of a user interface (UI). A UI may be used to provide guidance to the DP indicating an oral location for each next stimulus to be applied, for instance by audio-visual means. The UI may prompt for confirmation of each stimulus location via the UI, for instance by speech, gesture control, or with a user control function integrated in the probe, e.g. haptic feedback or a button on the probe.

The system in this embodiment further comprises means to measure the patient's pain response to the provided stimulus with physiological measures. This includes a biological or physiological signal sensing apparatus. There are multiple possibilities for this. One example is a skin conductance measurement apparatus, for example a galvanic skin response (GSR) apparatus.

A further example is a heart rate sensing means. Heart rate increases in response to pain. In addition, there is a reduction of heart rate variation with pain. Therefore one or both of both heart rate and/or heart rate variation (HRV) may be used as the biological/physiological signal in some embodiments. For heart rate, pain causes an upward spike (an elevated peak in the heart rate signal). For HRV, pain causes a downward spike in the HRV signal.

Another example is detection of facial expressions, which correlate with pain response, e.g. grimace, clench etc. A camera could be used to acquire image data of the face, and a facial analysis algorithm used to detect variations in facial expression.

Another example is measurement of electrical activity in different groups of muscles.

Another example is tracking of pupil size or movement of eyelids, e.g. by means of a camera.

Another example is measurement of blood flow, where blood flow increases responsive to pain/stress.

Another example is measurement of blood pressure, where blood pressure increases responsive to pain/stress.

In this embodiment, the system further comprises a processor adapted to determine a health state indicator for each location in the mouth based on the pain response measured for the given location. A digital record is further created of the health state indicator as a function of location. To determine the health state indicator, an algorithm is applied which encompasses a predefined relation between the (biological signal derived) pain level and the oral health indicator, e.g. periodontitis severity. This relation may be embodied by a simple relative relationship (e.g. a linear or non-linear function which relates the two). Alternatively, it may be embodied in a model encompassing multiple algorithms or in a machine learning model which has been trained using training data. With respect to the machine learning model, the training data used during the training procedure may comprise training data entries comprising example previous biosignal readings for a subject spanning a particular time window, or a pain level indicator derived from the biosignal response. These would be tagged with a ground truth indicative of the health state indicator value for that biosignal or that pain response.

The processor may be further adapted to generate a visual representation of the digital record of the health state indicator as a function of location. For example, this may comprise a visual map of the mouth, with health state indicator for each location marked on the map, e.g. with an overlaid color filter or textual marking. Different severities of a particular oral disease, as indicated by the health state indicator, might be shown via different colors, or different shades of the same color or other visual indicia. The visual representation may be output to a user interface for display to the dental practitioner.

In some examples, the visual mouth map may provide an indication simply of the pain level as a function of location in patient's mouth, which is sufficient to indicate possible problem areas to the DP. In other examples, the mouth map may provide an indication of the health state indicator as a function of location. Additionally, or alternatively, when the procedure is repeated at multiple points over time, a mouth map for (relative) disease progression over time may be constructed. For example, increased pain level is indicative of increased periodontitis severity, and determination of an absolute level of periodontitis may be less important than the trends over time.

In some examples, additional functionality may be provided to improve reliability of the pain response measurements and/or the reliability of the determined health state indicator for each location.

In particular, in some examples, a baseline measurement of the biological signal may be obtained prior to beginning the examination or dental procedure. This might be obtained with the patient resting, and based on determining an average level of the biosignal over a pre-defined time-window. For example, for skin conductance, the baseline level is indicative of the sweat gland reactivity due to the patient's normal arousal state. For instance, when a patient is more stressed during the clinical procedure, skin conductance level at baseline will be higher resulting in a higher reactivity. This can then be taken into account when determining pain responses, so that underlying stress response is not confused for pain response.

In some examples, a new baseline measurement of the biological signal may be obtained immediately subsequent to each pain response event in the biological signal, or subsequent to each applied stimulus. For example, after a stimulus, and consequent spike (up or down) in the biological signal, the biological signal may not return all the way down to its previous level, or may take some window of time to do this. If stimuli are being provided in fairly quick succession, the original baseline may no longer be a true indication of the real-time baseline. Acquiring a new baseline measurement after each stimulus, or after each pain response event in the biological signal allows to re-calibrate the baseline.

To describe this in another way, the decrease of a biological signal after pain depends upon the time between successive stimuli and the recovery capacity of the physiological system. If the time is not sufficient for the physiological system to recover after a first stimulus, the initial starting baseline measurement (representative of the physiology at rest) is not a good comparison to determine the pain of subsequent stimuli (e.g. due to a stack effect). The new background measurement may then serve as a new comparison. The benefits of this include: an accurate comparison value (baseline) even when the time interval between stimuli is not sufficient for physiology to recover; and that the baseline is made person-specific, thereby accounting for natural variation in the resting baseline and the physiological recovery rate between individuals.

In some examples, a maximum level of the biological signal (e.g. GSR reactivity) during treatment may be recorded, and this may be used as a control parameter to control for subject-specific differences in physiological responses. For example, the physiological response event peaks/drops may be normalized according to this parameter, and the corresponding pain level determinations normalised accordingly.

In some examples, demographic characteristics of the subject such as age, gender, or prior health status may be used as factors in the determination of the health state indicator for each location.

The reliability of the health state indicator determinations is aided by the standardization and reproducibility of the stimulus. However, there remains the possibility that the user may inadvertently introduce some bias into the measurements. Thus, the system may include functionality in some examples for detecting and flagging unintentional stimulus bias. Means may optionally be provided to correct for the stimulus bias. This can be achieved through processing applied by the processor.

For instance, in one example, the processor may be adapted to check for asymmetry in the pain measurements and/or health state indicator measurements between the left and the right-hand side of the mouth. For instance an average of the measurements for the left and right sides may be compared with one another. If there is a higher average response on one side compared to the other (e.g. with the difference exceeding some threshold) this may indicate some measurement bias (for example a right handed DP may find accessing one side of the mouth easier than the other).

Additionally or alternatively, the processor may be adapted to check for an asymmetry between the teeth of the upper and lower jaw. Again, if there is a clearly higher response on one jaw compared to the other this may indicate some measurement bias (for example the DP may find accessing the lower jaw easier than the other and hence applies a more controlled stimulus).

Additionally or alternatively, the processor may be adapted to check for a systematic trend upward or downward in the pain response over the course of the examination, (e.g. a steadily increasing score). Again such a pattern may indicate bias in providing the stimulus such that it is not standardized.

In any of the above cases, a further step in the processing algorithm may include correction of the biological signal, or the pain response measures, or the derived health state indicators to remove the artificial bias. For instance, where the bias is an underlying systematic trend, this might be removed through a filter (e.g. high pass filter). More generally, background removal methods are known in the art and the skilled person will understand how to apply these to correct for detected biases in the data.

A second example embodiment of the invention provides a system or device for home use by the patient. In some examples, this may comprise an oral care device, e.g. a cleaning device, which has the functionality for obtaining the pain responses integrated therein. This device may also be adapted for generating the standardized physical stimuli, and these stimuli may be combined with the normal oral care functionality so that the user can acquire the relevant data automatically while operating the device normally.

In one example, a system may be provided comprising a brushing mouthpiece device, and a biological signal sensor (e.g. a skin conductance sensor or PPG sensor), and wherein both are adapted to communicatively couple with a mobile device belonging to the user such as a smartphone. The smartphone may have a dedicated app installed. The mouthpiece provides (mechanical) stimuli at known locations in the mouth (see further below), and pain response is simultaneously measured with the biological sensor. The biological sensor may be integrated in a handle of the mouthpiece device. Alternatively, the biological sensor may be an auxiliary sensor. It may be integrated in a secondary wearable device, such as a smart watch. The smartphone receives the acquired measurement data and is adapted to perform the methodology described previously to determine the health state indicator information for each mouth location, and preferably generate a visual mouth map of for instance degree of periodontitis, and visually display this to the user.

This approach can have several advantages:
For the user, determining objectively the location of a pain is beneficial to improve oral health care behavior. However, for the user it is difficult to localize pain as all pain sensation in the mouth travels through the same nerve, i.e., the trigeminal nerve. Thus, the system helps the user to identify the source of pain by measuring the relative pain levels at different oral sites.

In some examples, the acquired information may also be sent to a computer or server of the DP for further assessment or for use in treatment planning.

The device can also be useful for monitoring recovery after a dental procedure or clinical treatment.

In some examples, the app may be configured to receive at the mobile device personalized OHC behavior tips transmitted by the DP, which may ultimately reduce chair time.

Instead of using a brushing mouthpiece device, a powered toothbrush device may be used as the oral care device. This could have skin conductance electrodes integrated in the handle for example.

Other options for delivering the mechanical stimuli may be as follows.

One embodiment comprises an oral tool which comprises a probe portion for insertion in interdental spaces, and/or for application on interdental papilla. For example the probe portion may comprises a fork-like member with at least two prongs. The tool may include means for driving vibration of the probe portion. In use, the vibrating or force-exerting fork-like tool is placed by a user on top of interdental papilla or inserted into the interdental space. The fork may be controlled to vibrate with a well-defined harmonic. It may generate a tapping or poking motion to enable periodic tooth contact with either fixed force or fixed excursion, where excursion means dragging or rubbing the tool along a defined excursion path. Additionally or alternatively, it may be adapted to poke the gums at the height of the interdental papilla, either with fixed force or fixed excursion.

In some examples, the same type of tool or a different tool could be provided, adapted to exert a well-controlled tapping motion. This could also be integrated in/delivered by a powered toothbrush (PTB).

In some examples, the provided tool may comprise an interdental brush, and wherein the mechanical stimuli are provided by inserting the brush into the interdental space. Instead of an interdental brush, a powered flossing tool might be used, with the flossing member inserted in the interdental space. In either case, the tool may comprise a pressure sensor for sensing a pressure with which the brush or flossing member is inserted. It may provide feedback to guide a user in applying a standard reference force, e.g. using acoustic feedback.

In some examples, the provided tool may comprise a fluid flossing or oral irrigation device. This is adapted to administer a pressurized fluid pulse or jet. This pulse or jet can provide the fixed force stimulus in fixed direction (e.g. perpendicular to the gum).

For examples in which the tool is to be inserted into interdental spaces, optionally sensing means may be integrated in the tool for sensing or detecting interdental spaces, and insertion therein. It can be difficult to guide a small probing tip of the tooth into an interdental space, and sensing can aid in this. By enabling reliable detection of interdental spaces, it is easier to apply the correct standardized and reproducible fluidic pressure or flow stimulus at the correct angle. For example, a standardized and reproducible fluid stimulus can be provided when a toothbrush is oriented with the cleaning elements (e.g. bristles) at a certain brushing angle relative to the gum line or pocket (e.g. jetting into the pocket), or when a controlled/optimum brushing user motion is used.

By way of example, suitable means for implementing interdental space sensing have been described in detail in the documents: WO 2020/212248 A1 and EP3725263A1.

The biological signal may be indicative not only of pain but also non-pain psychological arousal states such as stress. Thus, to improve accuracy and reliability, in some examples the processor may be further configured with functionality to distinguish pain from non-pain responses in the biological signal.

In particular, in some examples, the processor may further be adapted to determine a temporal proximity between each physiological response event and an immediately preceding contact event (i.e. mechanical stimulus application), and to classify the physiological response event as a pain event only contingent on the temporal proximity meeting a pre-defined criterion, e.g. that the physiological response event occurs within a pre-defined time window of the contact event.

A spike in the physiological signal closely temporally correlated with occurrence of a contact event is an indication of a pain response (i.e. the physiological response has a causal relationship with the contact, so it is likely to be pain). On the other hand, a spike in the biological signal not closely temporally correlated with a contact event is more likely to be a generalized sympathetic response of the subject to the overall experience, e.g. a psychological stress response. By measuring the temporal proximity between a physiological response event and a preceding contact event, pain can be more reliably identified. Thus, this set of embodiments proposes use of temporal correlation between the mechanical engagement with the tooth or oral tissue, and the biological signal. The degree of correlation can be used to differentially classify a subject response as pain or non-pain arousal.

In one example, in addition to the biological signal, an indication of timings of each of the contact events is obtained. Means for doing this have been discussed earlier in this disclosure and, for brevity, will not be repeated.

If a physiological response event falls within a pre-defined time window following a contact event, the physiological response may be classified as a pain response, and if it falls outside of any said time window following any contact event, it may be classified as a non-pain arousal event (e.g. a psychological stress response). The time window may be approximately 2 seconds, for example, between 1 and 2 seconds in some examples. The length of the time window may however be configured as desired, to optimize sensitivity of the pain detection vs specificity of pain detection. Also, in some scenarios, the time correlation may be slightly offset, leading to an extended delay between the stimulus and the physiological response (e.g. 1-3 seconds, or 0-3 seconds) if the physical stimulus shows an associated latency time. An example of such a latency time is if the physical stimulus comprises a temperature difference from the skin, whereby the heat capacity of the skin causes a latency before the pain is felt.

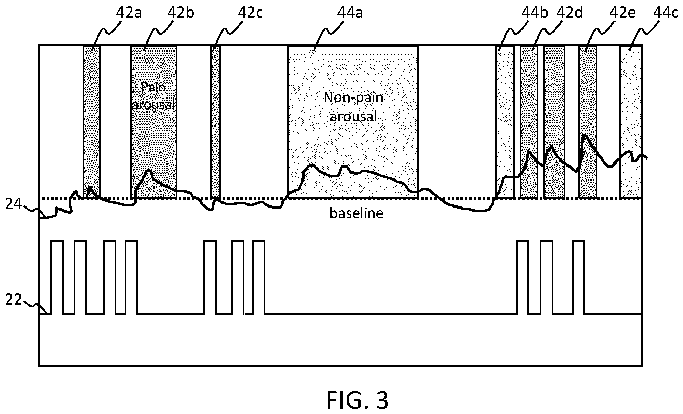

Fig. 3 schematically illustrates classification of physiological response events based on temporal proximity thereof to preceding contact events. A biological signal 24 as a function of time (x-axis) is shown along with a contact signal 22 as a function of time. The contact signal provides an indication of timings of the contact events. It might be received from a contact sensor for example, or from a user interface. In this example, the contact signal is a binary signal, having a value of 1 when contact is occurring and a value of zero when no contact is occurring. This leads to a series of square wave features in the signal, each representing a contact event. The biological signal is a multi-valued signal. The biological signal exhibits a plurality of physiological response events, where a physiological response event is characterized by a pre-determined signature or fingerprint in the signal having one or more characteristics. Most typically, a physiological response event may be identified as present between two time points of the biological signal where, between those two time points, the signal is above a pre-defined threshold value/height relative to a baseline level. Additionally or alternatively, there may be other conditions imposed on detection of a physiological response event, where these may include: a peak/drop in the signal of a pre-determined peak/drop height relative to a baseline level; a peak/drop of the signal exhibiting a pre-determined gradient on the leading edge of the peak/drop; a peak/drop of the signal having a pre-defined minimum area under the peak/drop; and/or a peak/drop of the signal exhibiting a pre-defined minimum sum along the leading edge of the peak/drop.

Fig. 3 shows the set of identified physiological response events 42, 44 in the biological signal, where some 42a-42e are characterized as pain response events, and some 44a-44c are characterized as non-pain arousal response events (e.g. physiological stress events).

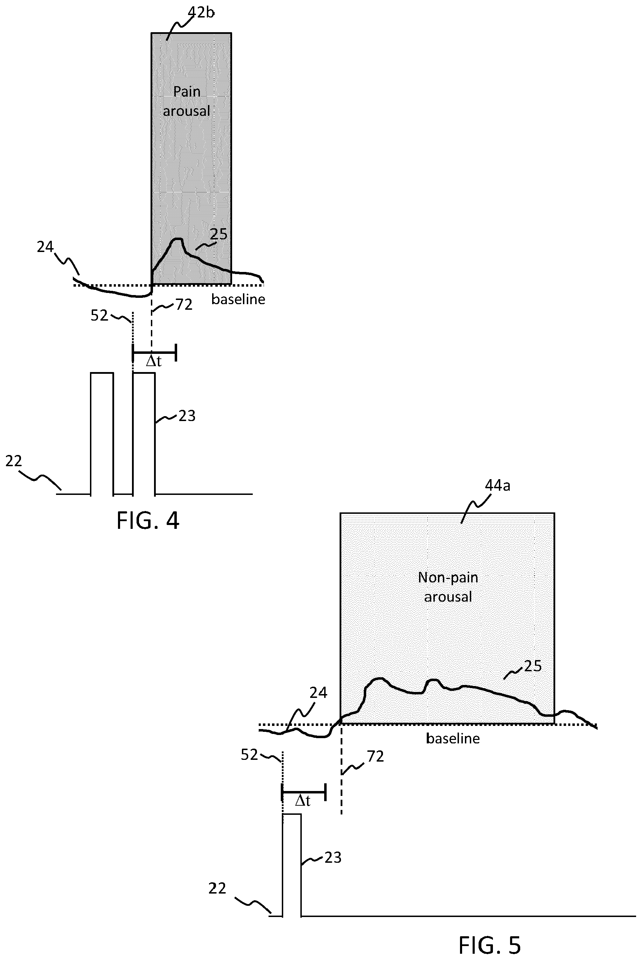

Fig. 4 schematically shows in more detail classification of one of the pain response events 42b of the data series of Fig. 3. The portion of the biological signal 24 and contact signal 22 immediately preceding and throughout the pain event are shown. A physiological response event 25 is identifiable in the biological signal 24 as a peak in the signal, which exceeds a pre-determined peak height. The onset time 72 of the physiological response event peak falls within a predetermined time window, Δt, extending from the onset time 52 of a preceding contact event 23. The physiological response event is therefore classified in this case as a pain event.

Fig. 5 schematically shows in more detail classification of one of the non-pain response events 44a of the data series of Fig. 3. The portion of the biological signal 24 and contact signal 22 immediately preceding and throughout the pain event are shown. A physiological response event 25 is identifiable in the biological signal 24 as a peak in the signal, which exceeds a pre-determined peak height. The onset time 72 of the physiological response event peak falls in this case outside of a predetermined time window, At, extending from the onset time 52 of any preceding contact event. In particular, it is outside of the time window, At, following the temporally closest contact event 23 preceding onset of the physiological response event. The physiological response event is therefore classified in this case as a non-pain arousal response, e.g. a stress response.

An aspect of the invention also provides a computer-implemented method for use in measuring a subject pain response to a standardized mechanical stimulus applied using an oral tool at a plurality of locations in a subject's mouth, and for assessing a pre-determined oral health indicator at the plurality of locations based thereon.

The method comprises receiving a biological signal of the subject; obtaining an indication of position of the oral tool for each of a plurality of mechanical stimuli applied; determining from the biological signal a subject pain response to each applied mechanical stimulus; determining using a classifier algorithm an oral health state indicator for each mouth location based on the pain response associated therewith; and generating a digital record comprising a representation of the oral health state indicator as a function of mouth location.

A further aspect of the invention also provides a computer program product comprising code means configured to cause a processor to perform the method outlined above, when the processor is operatively coupled with a biological signal sensing means for measuring a biological parameter of the subject, directly or indirectly indicative of a pain response of the subject to each mechanical stimulus.

As discussed above, various embodiments make use of a processor. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processor for use in measuring a subject pain response to a standardized mechanical stimulus applied using an oral tool at a plurality of locations in a subject's mouth, and for assessing a pre-determined oral health indicator at the plurality of locations based thereon, the processor adapted to:
receive a biological signal of the subject;
obtain an indication of an oral position of the oral tool for each of a plurality of mechanical stimuli applied;
determine from the biological signal a subject pain response to each applied mechanical stimulus;
determine an oral health state indicator for each mouth location based on the pain response associated therewith; and
generate a digital record comprising a representation of the oral health state indicator as a function of mouth location.

2. The processor of claim 1, wherein the mechanical stimulus comprises:
application of a standard reference force to an oral surface; and/or
probing a periodontal pocket to a standard reference depth.

3. The processor of claim 1 or 2, wherein the obtaining of the indication of the position of the oral tool for a given mechanical stimulus comprises:
receiving a positioning signal from a position tracking apparatus indicative of position of the oral tool;
obtaining an indication of a location indicated in a prompt delivered to a user by a user interface for prompting the user to manually apply the stimulus at the indicated location; or
receiving from a user interface a user-input indication of the position of the oral tool.

4. The processor of any of claims 1-3, wherein the biological signal comprises a skin conductance signal received from a skin conductance sensing apparatus, and optionally wherein the skin conductance sensing apparatus is a galvanic skin resistance measurement device.

5. The processor of any of claims 1-4, further adapted to generate user feedback via control of a user interface device operatively coupled to the processor, the user feedback comprising a visual representation of the oral health state indicator as a function of mouth location.

6. The processor of any of claims 1-5, wherein the processor is adapted to access a health record datastore, and to append the generated digital record of the oral health state indicator as a function of mouth location to the datastore, associated with subject identification information, and associated with a time-stamp.

7. The processor of claim 6, wherein the processor is adapted to access the health record datastore, to access historical records stored in the datastore for the subject of the oral health state indicator as a function of mouth location, and to compare the determined health state indicator for one or a plurality of the mouth locations with the historical values; and
optionally wherein the processor is adapted to generate longitudinal trend information for the oral health state indicator for one or a plurality of locations in the mouth and to output the trend information to a user interface for presentation to a user.

8. A system comprising:
an oral tool for use in applying a standardized mechanical stimulus to an oral location in the mouth of a subject, wherein the tool includes indicator or feedback means for guiding a manual application of the stimulus by a user, or the tool includes an actuation mechanism for automatically applying the stimulus; and
a means for measuring a biological signal directly or indirectly indicative of a subject pain response to each applied stimulus.

9. The system of claim 8, further comprising a processor adapted to obtain an indication of the subject pain response to each of a plurality of mechanical stimuli applied using the oral tool at different locations in the mouth over a session, and to determine an oral health state indicator associated with each pain response, and to generate a digital record thereof; and
optionally wherein the processor is further adapted to obtain an indication of a mouth location associated with each pain response and wherein the digital record represents the oral health state indicator as a function of mouth location.

10. The system of any of claims 8-9, wherein the oral tool is a dental probe for probing a periodontal pocket with a probe tip, and wherein the indicator or feedback means comprises a probing depth indicator which provides mechanical or visual feedback to an operator of the probe when the probe tip has been inserted to a predetermined depth in the pocket.

11. The system of claim 10, wherein the probing depth indicator comprises a ridge or outward protrusion from a surface of the probe tip, at a height from a terminal distal point of the probe tip corresponding to the pre-determined depth in the pocket to be probed.

12. The system of any of claims 8-9, wherein the oral tool comprises a dental probe for probing a periodontal pocket with a probe tip, and wherein the dental probe comprises a resilient element physically coupled with the probe tip for providing a resistive force feedback indicative of when a force with which the probe tip is being applied to an oral location by a user is equal to a pre-determined reference force.

13. The system of any of claims 8-12, wherein the oral tool comprises a probe portion for insertion in interdental spaces, and/or for application on interdental papilla, and optionally wherein the probe portion comprises a fork member with at least two prongs.

14. A computer-implemented method for use in measuring a subject pain response to a standardized mechanical stimulus applied using an oral tool at a plurality of locations in a subject's mouth, and for assessing a pre-determined oral health indicator at the plurality of locations based thereon, the method comprising:
receiving a biological signal of the subject;
obtaining an indication of position of the oral tool for each of a plurality of mechanical stimuli applied;
determining from the biological signal a subject pain response to each applied mechanical stimulus;
determining an oral health state indicator for each mouth location based on the pain response associated therewith; and
generating a digital record comprising a representation of the oral health state indicator as a function of mouth location.

15. A computer program product comprising code means configured to cause a processor to perform the method of claim 14 when the processor is operatively coupled with
a biological signal sensing means for measuring a biological parameter of the subject, directly or indirectly indicative of a pain response of the subject to each mechanical stimulus.
